# EUROPÄISCHE PATENTANMELDUNG

(11) **EP 0 819 930 A2**
(43) Veröffentlichungstag der Anmeldung: **21.01.1998**
(21) Anmeldenummer: 97111453.3
(22) Anmeldetag: 07.07.1997
(51) Int. Cl.: G01N 15/14, C12M 1/34

(54) **Verfahren und Vorrichtung zum Screening von Molekülen bezüglich ihres individuellen Bindungsverhaltens zu mindestens einem vorgegebenen Ligand**

(30) Priorität: 19.07.1996 DE 19629141
(71) Anmelder: BAYER AG, 51368 Leverkusen (DE)
(72) Erfinder: Luttermann, Klaus, 53819 Neunkirchen (DE); Diessel, Edgar, Dr., 50679 Köln (DE); Kosch, Winfried, Dr., 65203 Wiesbaden (DE); Weichel, Walter, Dr., 51519 Odenthal (DE)

(57) **Zusammenfassung**

Das Verfahren dient zum Screening von Molekülen aus Molekülbibliotheken bezüglich ihres individuellen Bindungsverhaltens zu mindestens einem vorgegebenen Ligand. Zu diesem Zweck werden die mit einem Fluoreszenzfarbstoff markierten Liganden mit der in Form einer Suspension vorliegenden Molekülbibliothek gemischt. Die Mischung wird nach Auswaschung der überschüssigen, nicht gebundenen Liganden auf ein zweidimensionales Substrat (2) ausplattiert. Anschließend werden in einem Fluoreszenzmikroskop (5) die örtlichen Fluoreszenzintensitäten auf dem Substrat elektrooptisch erfasst und nach vorgegebenen Selektionskriterien elektronisch diskriminiert. Die auf diese Weise selektierten und lokalisierten Objekte werden danach sequentiell durch eine vom Bildrechner koordinatengesteuerte Verschiebung zwischen dem Substrat (2) und einem Separationsaktor (20,21) ortsgenau positioniert und durch den Separationsaktor (20,21) vom Substrat (2) örtlich getrennt.

## Beschreibung

Die Erfindung betrifft ein Verfahren und eine Vorrichtung für eine optimierte Selektion von Molekülen aus Molekülbibliotheken hinsichtlich ihres Bindungsverhaltens gegenüber einem oder mehreren vorgegebenen Targetmolekülen, das erstmals eine sehr große Vielfalt von Molekülen zu untersuchen gestattet, Auskunft über individuelle Bindungsereignisse gibt und eine schonende Selektion der zu selektierenden Moleküle erlaubt.

Die Molekülbibliotheken als Basis für das erfindungsgemäße Selektionsverfahren werden durch chemische Methoden (kombinatorische Chemie) oder durch biotechnologische Methoden im Rahmen der angewandten molekularen Evolution (AME) generiert. Die kombinatorische Chemie nutzt dabei verschiedenartigste chemische Reaktionen zum Aufbau von Molekülbibliotheken, wahrend die AME mit Hilfe von Mutationsstrategien eine große Population von unterschiedlichen Biopolymeren erzeugt. Die effiziente Selektion von an ein spezifisches Target bindende Moleküle bzw. Biopolymere ist ein wesentlicher Bestandteil zum Auffinden neuer pharmazeutischer Wirkstoffe (Leitstrukturen) und daher von großer Bedeutung.

Grundsätzlich sind Verfahren im Rahmen der AME zum Protein-Design bekannt und etabliert. Anwendung fand die evolutive Strategie z.B. in dem Auffinden von Peptid-Liganden (O'Nell et al., Prot. 14, 509 (1992)) und der Entwicklung hochaffiner maßgeschneiderter Antikörper (Breitling et al., Gene 104, 147 (1991), Clackson et al., Nature 352, 624 (1991), Marks et al., J. Mol. Biol. 222, 581 (1991), Persson, Proc. Natl. Acad. Sci. USA 88, 2432 (1991)). Insbesondere das Antikörper-Engineering stellt ein vielversprechendes Konzept der angewandten evolutiven Biotechnologie dar. Aufgrund ihrer hochselektiven Bindungseigenschaften sind Antikörper bedeutende Reagenzien in der Forschung, Diagnostik und Therapie (Plückthun, J. Anal. Chem. 337, 13 (1990), Plückthun, Biotechnology 9, 545 (1991), Little et al., Biotech. Adv. Vol. 12, 539 (1994)).

Neben der Bildung von Molekülbibliotheken durch die AME hat sich in letzter Zeit vor allem die Nutzung der kombinatorischen Chemie etabliert, die unterschiedlichste chemische Reaktionen zur Synthese der Bibliothek auf festen Trägern wie z.B. der von Polymerbeads ausnutzt (D.J. Ecker, S.T. Crooke, Biotechnology, 1995, 13, 351; R.M.J. Liskamp, Angew. Chemie, 1994, 106, 661; T. Carell, E.A. Winter, A. Bashir-Hashemi, J. Rebek, Angew. Chemie, 1994, 106, 2159; J.W. Metzger, K.-H. Wiesmüller, V. Gnau, J. Brünjes, G. Jung, Angew. Chemie, 1993, 105,901). Exemplarisch sei hier die Festphasensynthese von Benzodiazepinen durch Hobbs De Witt et al. (Proc. Natl. Acad. Sci. U.S.A., 1993, 60, 6909) erwähnt.

Als Voraussetzung für das erfindungsgemäße Verfahren müssen die Molekülbibliotheken trägerbasiert vorliegen. Damit wird eine Messung an einer großen Zahl gleicher Moleküle ermöglicht. Auf diese Weise kann das individuelle Bindungsverhalten ausreichend charakterisiert werden. Als trägerbasiertes System kommen z.B. Bakterien im Rahmen der AME oder Polymerbeads im Rahmen der kombinatorischen Chemie in Frage.

In der Literatur sind Verfahren zur Darstellung von Proteinbibliotheken auf E. coli Bakterien (Hofnung, Meth. Enzymol. 134, 77 (1991), Klauser et al., EMBO J. 9, 1991 (1990), Fuchs et al., Biotechnology 9, 1369 (1991), Francisco et al., Proc. Natl. Acad. Sci. USA 89, 2713 (1992), Pugsley, Proc. Natl. Acad. Sci. USA 89, 12058 (1992)) bekannt. Darüberhinaus wird die Expression auf einer Phagenoberfläche (Hoogenboom et al., Immunolog. Reviews 130, 41 (1992)) beschrieben, die aufgrund der Phagengröße (<< 1 µm) für das erfindungsgemäße Verfahren irrelevant ist.

Technische Lösungen für Selektionsverfahren zur Separierung von Zellen aus einer großen Zahl (> 10⁶) sind mit dem FACS (Fluorescence Activated Cell Sorter) und MACS (Magnetic Activated Cell Sorter) kommerziell erhältlich.

Bei der fluoreszenzaktivierten Zellsortierung kommen elektrostatische Prinzipien zur räumlichen Separation zum Einsatz. Ein kommerzieller FACS (Becton & Dickinson: FACStar plus) ist in der Lage, pro Tag ca. 10⁸ Zellen sequentiell zu prozessieren. Hierbei ist aber damit zu rechnen, daß die nutzbare Sortierrate deutlich unter 100 % liegt. Bei sehr seltenen Ereignissen in einer Zellpopulation sind jedoch hohe Durchsätze wünschenswert von etwa 10⁹ Zellen.

Im Vergleich zum erfindungsgemäßen Verfahren findet beim FACS zudem der Sortierprozeß unmittelbar nach dem Meßprozeß statt, so daß bei einer nachträglichen Korrektur der Schwellwerte für Affinitäten der Sortierprozeß als ganzes wiederholt werden müßte. Damit geht eine weitere mechanische Belastung des Molekülträgers bzw. der Bakterien einher.

Bei dem MACS-Sortierverfahren wird die Bindung der relevanten Zellen an magnetische Beads ausgenutzt. Im Separationsschritt werden die in dieser Weise markieten Zellen in der MACS-Säule durch ein inhomogenes Magnetfeld zurückgehalten, während die nichtmarkierten Zellen ungehindert die Säule passieren. Hierbei kann jedoch nur zwischen magnetischen und nichtmagnetischen Zellen unterschieden werden. Damit erlaubt der MACS eine schnelle Bearbeitung großer Populationen, doch lassen sich keine Informationen über individuelle Bindungsereignisse gewinnen.

Die Separation von Beads in der kombinatorischen Chemie wird üblicherweise in einem manuellen Prozeß durchgeführt.

Der Erfindung liegt die Aufgabe zugrunde ein Verfahren zu entwickeln, mit dem aus einer großen Anzahl (10⁹) von Molekülen aus einer Molekülbibliothek einzelne Objekte innerhalb von 24 h detektiert und separiert werden, die durch ihre speziellen Bindungsaffinitäten an einen oder mehrere vorgegebene Liganden gekennzeichnet sind. Für die anschließende Vermehrung (Amplifikation) von Bakterien im Rahmen der AME, soll die Vitalität der Bakterienpopulation erhalten bleiben.

Diese Aufgabe wird erfindungsgemäß durch ein Verfahren mit folgenden Schritten gelöst:
a) Die zu bindenden Liganden werden mit einem Fluoreszenzfarbstoff markiert und mit der in Form einer Suspension vorliegenden Molekülbibliothek gemischt.
b) Die überschüssigen, nicht an Moleküle der Molekülbibliothek gebundenen Liganden werden danach ausgewaschen und entfernt.
c) Diese Mischung wird auf ein zweidimensionales Substrat ausplattiert.
d) Das so beschichtete Substrat wird in ein Fluoreszenzmikroskop gebracht. Anschließend werden die auf dem Substrat beobachteten örtlichen Fluoreszenzintensitäten elektrooptisch erfasst und mit Hilfe einer CCD-Kamera als Gesamtbild oder schrittweise in Form von Teilbildern digital erfaßt und nach vorgegebenen Selektionskriterien in Form von Schwellwerten elektronisch diskriminiert, wobei die auf dem Substrat befindlichen, durch eine hohe Bindungsaffinität der Liganden an Moleküle der Molekülbibliothek gekennzeichneten und damit die Selektionskriterien erfüllenden Objekte erkannt und durch Abspeicherung in einem Bildrechner lokalisiert werden.
e) Die so selektierten Objekte werden dann sequentiell durch eine vom Bildrechner koordinatengesteuerte, relative Verschiebung zwischen dem Substrat und einem Separationsaktor in den Arbeitspunkt des Separationsaktors positioniert, vom Substrat entnommen und örtlich getrennt abgelegt.

Der verfahrensgemäßen Identifikation und Separation von Molekülen aus einer Biomolekül-Bibliothek geht voraus, daß diese Moleküle in ausreichender Anzahl auf der Oberfläche einer geeigneten biologischen Zelle dargestellt werden. Hierzu wird die genetische Information für ein Biomolekül in eine große Population von Mikroorganismen eingeschleust, welche daraufhin das Biomolekül synthetisieren. Die Verfahren, welche diese Steuerung eines Mikroorganismus zur biochemischen Synthese beinhalten, werden unter dem Begriff Expressions-Systeme zusammengefaßt. Die Vielfalt der Bibliothek entsteht durch Mutantenvielfalt der eingeschleusten Information. Dabei können Komplexitäten > 10⁹ erzeugt werden.

Vorteilhaft erfolgt die elektrooptische Erfassung der örtlichen Fluoreszenzintensitäten auf dem Substrat mit Hilfe einer CCD-Kamera in Form eines Gesamtbildes oder schrittweise in Form von Teilbildern. Das Gesamtbild oder die Teilbilder werden dann, gegebenenfalls nach einer Datenreduktion, dem Bildrechner zur Abspeicherung und zur bildanalytischen Weiterverarbeitung und Auswertung nach den vorgegebenen Selektionskriterien zugeleitet.

Alternativ kann die elektrooptische Erfassung der örtlichen Fluoreszenzintensitäten auf dem Substrat auch mit Hilfe eines Laser-Scanners erfolgen, dessen Ausgangs-signale nach den vorgegebenen Selektionskriterien elektronisch bewertet und in Verbindung mit den zugehörigen Ortskoordinaten dem Bildrechner zugeführt werden.

Vorzugsweise werden bei der Bereitstellung einer Biomolekülbibliothek als bakterielles Expressionssystem E. coli Bakterien verwendet.

Besonders bevorzugt werden zur Bereitstellung einer Peptid- und/oder Proteinbibliothek als spezielles bakterielles Expressionssystem E. coli Bakterien verwendet, die durch genetische Manipulation Variationen von LamB-Transmembranproteinen exprimieren.

Neben einer optimierten Selektion und Separation von Molekülen aus einer Bibliothek, die von Mikroorganismen gebildet werden, betrifft das Verfahren auch die Selektion und Separation von Molekülen aus einer mittels chemischen Methoden hergestellten Molekül-Bibliothek. In der kombinatorischen Chemie werden Molekülbibliotheken an einer festen Phase mittels chemischer Methoden (Reaktionen) erzeugt. Die Vielfalt der Bibliothek entsteht dabei durch die Nutzung unterschiedlichster Reaktionen und Reagenzien. Diese Molekülbibliotheken werden vorteilhaft auf polymeren Beads dargestellt. Vorzugsweise werden als polymere Träger Kunststoffbeads auf Polystyrol- oder Polyacrylamidbasis mit einem Durchmesser von 50 µm bis 200 µm eingesetzt.

Um mehrere Liganden gleichzeitig untersuchen zu können, werden zweckmäßig verschiedene Liganden mit unterschiedlichen Fluoreszenzfarbstoffen markiert. Für die verschiedenen Liganden werden die Selektionskriterien in Form einer Liste mit den Schwellwerten für die verschiedenfarbigen Fluoreszenzintensitäten vorgegeben.

Eine Weiterentwicklung des erfindungsgemäßen Verfahrens besteht darin, daß die die Molekülbibliothek tragenden Proteine auf den Bakterien oder die Verbindungen aus der kombinatorischen Chemie auf den Polymerbeads mit einem weiteren Fluoreszenzfarbstoff markiert werden und die Fluoreszenzintensität der Bindungsstellen als zusätzliches Selektionskriterium bei der Bildauswertung berücksichtigt wird.

In diesem Fall kann das Verhältnis der für den Liganden charakteristischen Fluoreszenzintensität und der für die Bindungsstelle charakteristischen Fluoreszenzintensität als ein für die Bindungsaffinität maßgebliches Selektionskriterium bei der Bildauswertung benutzt werden.

Die Vorrichtung zur Durchführung des erfindungsgemäßen Verfahrens weist ein inverses Fluoreszenzmikroskop und einen Verschiebetisch zur Halterung eines Trägers mit der ausplattierten Objektsuspension, sowie mindestens eine Lichtquelle zur Fluoreszenzanregung auf und ist erfindungsgemäß dadurch gekennzeichnet,
a) daß eine aus einer CCD-Kamera oder aus einem Laser-Scanner bestehende elektrooptische Abtasteinrichtung zur Erfassung der örtlichen Fluoreszenzintensitäten der auf dem Substrat ausplattierten Bakteriensuspension vorgesehen ist, die mit einem Bildrechner zur Weiterverarbeitung und Speicherung der Fluoreszenzbildinformation verbunden ist,
b) daß oberhalb des Verschiebetisches ein Separationsaktor zum Transfer der selektierten Objekte angeordnet ist,
c) und daß der Bildrechner die Positionierung des Verschiebetisches relativ zum Separationsaktor steuert.

Der Separationsaktor besteht dabei vorteilhaft aus einer in einen Mikromanipulator eingebauten, absenkbaren Mikrokapillare.

Gemäß einer bevorzugten Ausführungsform wird dem Substrat das Fluoreszenzanregungslicht über mindestens einen vom Mikroskop-Strahlengang getrennten Lichtleiter zugeführt. Zu diesem Zweck sind zwei verschiedenene, in denselben Lichtleiter einkoppelbare Laser mit verschiedenen Wellenlängen zur Fluoreszenzanregung vorgesehen.

Zur Vermeidung von Artefakten wird das Fluoreszenzmikroskop einschließlich der Beleuchtungseinrichtung und der Mikrokapillare in eine Klimakammer eingebaut wird, die durch einen wasserdampfgesättigten Luftstrom auf Temperaturen zwischen 1°C und 10°C gekühlt wird. Solche Artefakte können z.B. die Vermehrung der Bakterien, Schrumpf des Agarosesubstrates und Temperaturschwankungen sein.

Der Hauptvorteil des beschriebenen Verfahrens besteht in der Einzelquantifizierung und -selektion von Bindungskomplexen bei einem gleichzeitig hohen Durchsatz der zu analysierenden Bindungsereignisse. Dies führt zu einer erheblichen Reduktion des Zeitaufwandes und der Kosten. Bei biologischen Objekten wird zudem die Vitalität der selektierten Zellen gewahrt.

Im Rahmen grundlagenwissenschaftlicher Untersuchungen kann das Verfahren zur Charakterisierung von Molekülbibliotheken eingesetzt werden. Für einen industriellen Einsatz kommt die Leitstruktursuche für neuartige Pharmaka in Frage. Mit Hilfe des erfindungsgemäßen Verfahrens ist bei Etablierung geeigneter Antikörperbibliotheken eine pharmakologische Individual-Therapie möglich, bei der ein maßgeschneidertes Medikament für den Einzelpatienten an einem Tag gefunden isoliert und vermehrt werden kann.

Im folgenden wird die Erfindung an Hand von Zeichnungen und Ausführungsbeispielen näher erläutert. Es zeigen:
- Fig. 1: ein planares Substrat mit der ausplattierten Bakteriensuspension
- Fig. 2: schematisch eine Selektionsapparatur auf der Basis einer CCD-Kamera
- Fig. 3: schematisch eine Selektionsapparatur auf der Basis eines Laser-Scanners
- Fig. 4: einen Separationsaktor zum Transfer (Trennung) der selektierten, charakteristischen Objekte

Die Durchführung des erfindungsgemäßen Selektions- und Separationsverfahrens zur Selektion und Separation von Molekülen mit hoher Bindungsaffinität zu einem Targetmolekül erfordert die Bereitstellung einer geeigneten Molekülbibliothek und ein Targetmolekül, das im folgenden als Ligand bezeichnet wird. Dies wird nachstehend am Beispiel einer Peptidbibliothek beschrieben. Für die Präsentation dieser Biomolekül-Bibliothek werden hier E. coli Bakterien verwendet, die durch genetische Manipulation Variationen von LamB-Transmembranproteinen exprimieren. Als Ligand wird der Acetylcholinrezeptor gewählt. Im Gegensatz zu der weitverbreiteten Phagenmethode bietet das Bakteriensystem aufgrund der Vielzahl gleichartiger Bindungsereignisse auf der Bakterienoberfläche den Vorteil, eine Einzelbetrachtung der Wechselwirkung zwischen peptidtragender Zelle und Rezeptor vorzunehmen. Ein analoges Vorgehen mit Phagen scheidet aufgrund der geringen Größe der Phagenpartikel aus.

Zu den peptidtragenden Bakterien werden Liganden gemischt, die mit einem Fluoreszenzfarbstoff markiert sind. Hierzu wird beispielsweise der Farbstoff ®Cy5 der Firma Biological Detection Systems eingesetzt, der bei 650 nm angeregt wird und bei 670 nm emittiert. Aufgrund der langwelligen Anregung kann das Fluoreszenzsignal weitgehend frei von Autofluoreszenz detektiert werden. Da die Anzahl der Peptide auf der Bakterienoberfläche nicht konstant ist, wird ein Molekülabschnitt, der bei sämtlichen Peptiden auf allen Bakterien gleich ist, spezifisch mit einem weiteren Farbstoff markiert, um mit dessen Fluoreszenzsignal ein Maß für die Anzahl der Bindestellen zu erhalten. Aus dem Verhältnis der beiden Fluoreszenzintensitäten kann ein Maß für die Bindungsaffinität des Komplexes angegeben werden. Als zweiter Fluoreszenzfarbstoff wird ®FITC der Firma Molecular Probes eingesetzt, das bei 490 nm angeregt wird und bei 520 nm emittiert. Mit diesen spektralen Charakteristiken werden die beiden Fluoreszenzsignale ohne gegenseitige Beeinflussung gemessen.

Nach dem Mischen des farbstoffmarkierten Liganden mit der Bakteriensuspension (Inkubation) werden die Bakterien durch Abzentrifugieren und anschließendes Dispergieren in reiner Pufferlösung gewaschen. Damit verbleiben in der Bakteriendispersion nur noch Fluoreszenzfarbstoffe zurück, die sich ausschließlich auf der Oberfläche der Bakterien befinden.

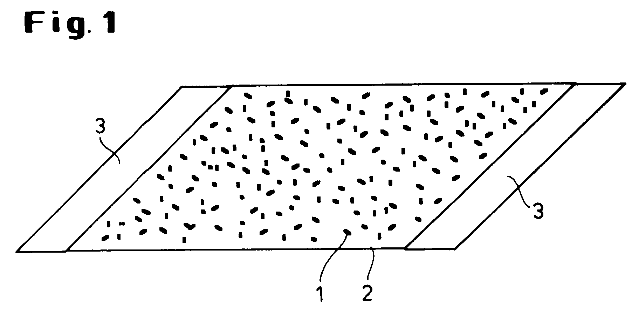

Diese Bakteriendispersion 1 wird sodann auf eine auf einem planaren Substrat 2 befindlichen Agaroseoberfläche mit einem Spatel ausplattiert (s. Fig. 1). Die Agaroseoberfläche ist in der Weise geteilt, daß sich im Randbereich jeweils getrennte Zonen 3 befinden, auf denen später die separierten Bakterien zu liegen kommen. Diese Agaroseoberflächen 3, die im Gegensatz zum Substrat Nährstoffe enthalten, lassen sich von dem Substrat 2 durch Abheben trennen. Zur anschließenden effizienten Vermessung der Fluoreszenzintensitäten der auf dem Substrat planar ausgebrachten Bakterien wird eine Flächenbelegungsdichte von 10 % angestrebt. Bei einer Population von 10⁹ Spezies ergibt sich daraus eine Gesamtfläche von 250 cm².

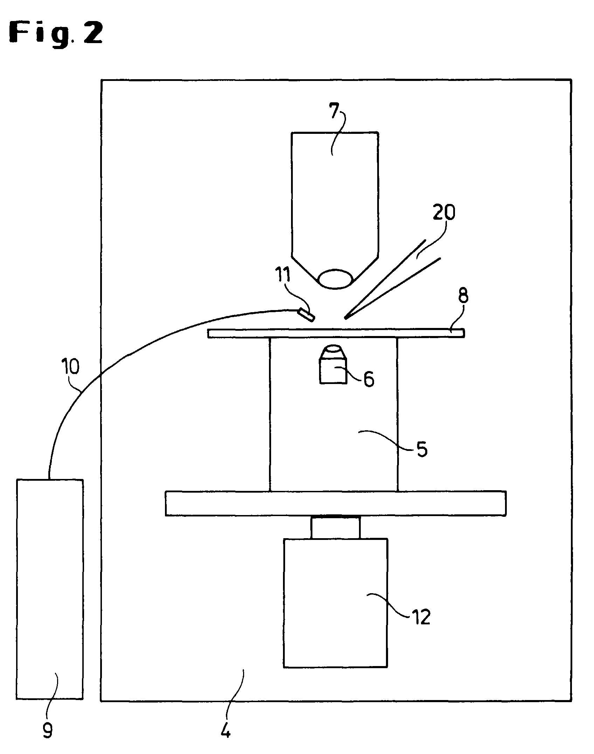

Fig. 2 zeigt schematisch den Aufbau der gesamten Selektionsmaschine. Die verfahrenstechnischen Aufgaben liegen in der Aufnahme von Fluoreszenzaufnahmen und der anschließenden Separation von Bakterien, die sich durch vorgegebene, spezifische Bindungseigenschaften auszeichnen. Während der Messung sollen Artefakte wie z.B. die Bakterienvermehrung ausgeschlossen werden. Dies wird durch eine Klimakammer 4 bewerkstelligt, die von einem wasserdampfgesättigten Luftstrom bei 4°C gekühlt wird. Der Kern der Maschine besteht aus einem inversen Mikroskop 5. Zu dem Mikroskop gehören das Abbildungsobjektiv 6, der Beleuchtungskondensor 7, sowie der Verschiebetisch 8, auf dem das Substrat 2 gehaltert wird. Als Lichtquelle für die Fluoreszenzspektroskopie wird ein Lasermodul 9 eingesetzt, das aus zwei Lasern und einer optischen Anordnung besteht, die die zwei Laserstrahlen über elektronische Verschlüsse alternierend in einen Lichtwellenleiter 10 mit einen Kerndurchmesser von 200 µm einkoppelt. Zur Anregung des Ligandenfluorophors kommt ein Kr-Ionengaslaser zum Einsatz, der bei einer Wellenlänge von 647 nm mit einer Leistung von 500 mW betrieben wird. Die zweite Lichtquelle für die Anregung des zweiten Fluorophors an der Bindungsstelle auf der Bakterie ist ein Ar-Ionengaslaser, der auf eine Emission bei 488 nm und einer vergleichbaren Leistung eingestellt wird. Am Ende der Faser ist eine Mikrooptik 11 angebracht, die auf der Probe einen Leuchtfleck von 1.5 mm Durchmesser erzeugt. Die externe Laseranregung vermeidet Streulicht und Hintergrundfluoreszenz im Abbildungsobjektiv 6. Die hohe Laserintensität erlaubt Belichtungszeiten pro Aufnahme im Bereich von 0.1 bis 1 s. Das von dem Substrat 2 emittierte Fluoreszenzlicht wird von einer gekühlten CCD-Kamera 12 detektiert. Die Pixelanzahl dieses Fluoreszenzbildesbeträgt 1000 * 1018, so daß bei einer 10-fachen Mikroskopvergrößerung mit einer Auflösung von 1.2 µm gerechnet werden kann. Dieser Wert ist auf die Bakteriengröße abgestimmt, die in diesem Bereich liegt. Vor der Kamera 12 befindet sich ein auf die beiden Fluorophore abgestimmtes Emissionsfilter. Über eine hier nicht dargestellte Einrichtung wird die Fokusposition des Objektivs 6 motorisch nachgeregelt. Eine Vorrichtung zu dieser Nachfokussierung, die mit einem Rückreflex einer Oberfläche arbeitet, ist z.B. in (G. Bouwhuis et al., p. 75ff, A. Hilger Ltd. (UK/USA), 1985)) beschrieben.

Zur effektiven Bildauswertung wird noch auf der Ebene des Frame Grabber Boards der CCD-Kamera eine Datenreduktion vorgenommen. Die Software ist hierbei auf die Detektion erwartungsgemäß seltener Bindungsereignisse ausgelegt. Unter Zuhilfenahme von Look up Tables (LUT) wird zunächst geprüft, ob auf der Fluoreszenzaufnahme der markierten Liganden Fluoreszenzsignale oberhalb einer gewählten Schwelle S₁ liegen. Bei einem positiven Bescheid wird die zweite Fluoreszenzaufnahme des Bindungsstellenfluorophors vorgenommen. Nach Berechnung des Quotienten aus den beiden Signalen werden nur die Pixelkoordinaten samt Quotient auf dem Bildrechner abgespeichert, bei denen diese Quotienten oberhalb einer gewählten Schwelle S₂ liegen. Hierzu werden auf dem Frame Grabber Board die Zeilen ermittelt, die signifikante Pixel enthalten. Im zweiten Schritt werden diese Zeilen auf den Bildrechner transferiert und dort nach den signifikanten Pixeln abgesucht. Alternativ kann aber auch die gesamte bildanalytische Auswertung des Ligandenbildes nach den vorgegebenen Selektionskriterien, d.h. die Erkennung und Lokalisierung der auf dem Substrat 2 befindlichen, durch eine hohe Bindungsaffinität der Liganden an die Moleküle der Molekülbibliothek gekennzeichneten Objekte, allein im Bildrechner erfolgen. Unter "hoher Bindungsaffinität" wird dabei verstanden, daß die Fluoreszenzintenstäten dieser Objekte die Selektionskriterien hinsichtlich des oben erwähnten Schwellwertes S₂ erfüllen.

Dem gesamten Prozeß der Fluoreszenzaufnahmen liegt die folgende Zeitbetrachtung zu Grunde. Bei einer 10-fachen Vergrößerung erfaßt die CCD-Kamera ein Bildfenster von 1.2 * 1.2 mm. Mit dem Flächenbedarf für alle Bakterien von 2.5*10⁴ mm² müssen 17400 Rahmen abgearbeitet werden. Pro Rahmen wird für die Auswertung einer Doppelfluoreszenz eine Zykluszeit von 4.1 s abgeschätzt, die eine jeweilige Belichtungszeit von 1 s einschließt. Die Zeit für die Bewegung des Verschiebetisches ist hierbei berücksichtigt. Dies führt zu einer Gesamtzeit für den kompletten Belichtungsvorgang von 20 h bzw. zu einem Bearbeitungsdurchsatz von 10⁹ pro Tag und entspricht damit der Vorgabe.

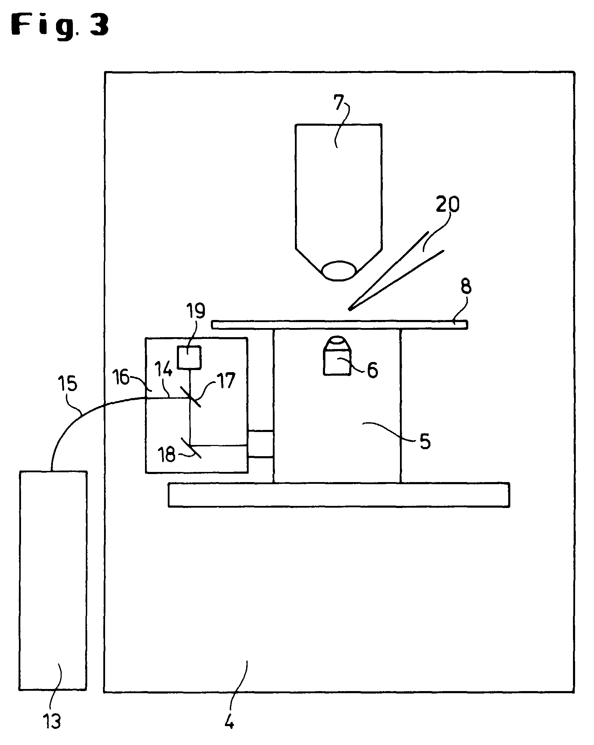

Alternativ zur Detektion fluoreszierender Objekte mit Hilfe einer simultanen Fluoreszenzanregung des gesamten Mikroskopgesichtsfeldes und der parallelen Detektion des Fluoreszenzbildes mit einer CCD Kamera kann auch eine punktweise abrasternde Fluoreszenzanregung mit einer Laser-Scan-Einrichtung gewählt werden. Ein derartiger Aufbau ist schematisch in Fig. 3 dargestellt. Der von einem Ar-Kr-Laser 13 mit einer Ausgangsleistung von 20 mW erzeugte Laserstrahl 14 wird über eine Monomode-Lichtleitfaser 15 in ein Optikmodul 16 eingekoppelt. In diesem Modul 16 wird der anregende Laserstrahl über einen dichroitischen Strahlteiler 17 auf einen x-y-Spiegel 18 geführt. Der computergesteuerte Spiegel 18 lenkt den Laserstrahl in der Weise ab, daß der über das Objektiv 6 zu einem Punkt fokussierte Laserstrahl die Probenebene in. x-y-Richtung abrastert. Die Ortskoordinaten eines fluoreszierenden Objekts werden hierbei über die Steuerparameter des Ablenkspiegels gespeichert. Hierbei sind diese Parameter in eindeutiger Weise mit dem x-y-Ort des Laserstrahls auf der Probenoberfläche verknüpft Diese Zuordnung wird in handelsüblichen Scannern ausgenutzt (z.B. Leica TCS4D Scanner, Leica Lasertechnik GmbH, Heidelberg, Deutschland). Das von der Probe ausgehende Fluoreszenzlicht gelangt über das Objektiv 6 und den Ablenkspiegel 18 auf den Strahlteiler 17 und wird hier im Gegensatz zum Anregungsstrahl transmittiert. Das Fluoreszenzlicht wird hinter dem Strahlteiler 17 in einem Detektionsmodul 19 nachgewiesen. Dieses Modul besteht aus einem Pinhole (Lochblende), mit dem Fluoreszenzlicht außerhalb der Fokusebene unterdrückt werden kann, zwei Photomultipliern für die zwei Fluoreszenzemissionen von FITC und Cy5 und den entsprechenden chromatischen Filtern. Während des Scanprozesses werden die Fluoreszenzintensitäten der markierten Liganden und auch des Bindungsstellenfluorophors simultan gemessen und durcheinander dividiert, so daß keine separate zweite Fluoreszenzaufnahme erforderlich ist. Damit kann der Quotient anhand eines Schwellwertes S₂ online diskriminiert werden kann und nur die Koordinaten und Intensitäten der Pixel stehen nach erfolgtem Scan im Arbeitsspeicher des Steuer- bzw. Auswerterechners, deren Intensitäten oberhalb der gesetzten Schwelle S₂ liegen. Alternativ dazu kann jedoch auch das gesamte Bild auf dem Auswerterechner einer Bearbeitung nach erfolgtem Scanprozeß unterzogen werden, um gegebenenfalls nachträglich mit verschiedenen Schwellwerten zu diskriminieren.

Bei einer Scanfläche von 2.5 * 10⁴ mm², einer Gesichtsfeldgröße von 1 mm², einer Scandauer von 1.1 s (Zeiss-LSM) pro Gesichtsfeld ergibt sich unter Berücksichtigung der Verschiebung des Probentisches eine Gesamtzeit von 11 h für die komplette Fluoreszenzanalyse.

Nach dem kompletten Scanvorgang der CCD-Kamera oder des Laser-Scanners liegt im Speicher des Bildrechners eine Liste der Ortskoordinaten von Bakterien mit spezifischen Bindungseigenschaften vor, mit deren Hilfe die Separation der Bakterien vorgenommen wird. Gegebenenfalls kann die Anzahl der zu separierenden Bakterien anhand der Liste eingeschränkt werden.

Nachdem das Screening abgeschlossen ist und die Ortskoordinaten der zu separierenden Bakterien bekannt sind, müssen diese mit einem geeignetem Separationsaktor von dem Substrat entfernt und auf einem Zielsubstrat abgelegt werden. Zu diesem Zweck wird ein auf einer Mikrokapillare 20 beruhender Separationsaktor eingesetzt. Derartige Separationsaktoren sind prinzipiell aus der Patch Clamp Technik bekannt.

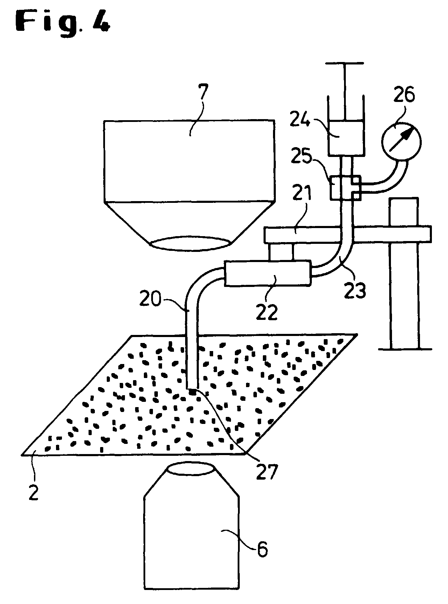

In Fig. 4 ist die Apparatur für den Transferprozeß schematisch dargestellt. Die mit Hilfe eines Mikromanipulators 21 verfahrbare Mikrokapillare 20 wird nun entsprechend der im Bildrechner vorliegenden Koordinatenliste nacheinander zu den zu selektierenden Bakterien gebracht und exakt oberhalb davon positioniert. Der Bildrechner fungiert also als Steuerrechner für den Mikromanipulator 21 zum Aufsuchen und zur Positionierung der Mikrokapillare 20 am Ort der ausgewählten Bakterien. Sodann wird die ausgewählte Bakterie mit der Mikrokapillare 20 von der Substratoberfiäche abgesaugt und auf einem Zielsubstrat wieder ausgebracht. Als Zielsubstrat dienen hier die Randzonen 3 auf dem Substrat 2, die mit Agar- bzw. Agaroseoberflächen präpariert sind. Der Durchmesser der Mikrokapillare wird aufgrund der angestrebten Manipulation einzelner Bakterien bei einer hohen Belegungsdichte an die Größe der Objekte angepaßt und hat demgemäß einen Durchmesser zwischen 2 und 20 µm.

Für den Transfer der Bakterien werden vorzugsweise Mikrokapillaren aus einem Spezialglas verwendet, die durch einen Ziehprozeß im geschmolzenen Zustand hergestellt werden. Für die zu beschreibenden Experimente werden Borosilikatglasröhrchen (Fa. Hilgenberg, Malsfeld, GER) mit einem Ausgangsdurchmesser von 1.6 mm eingesetzt. In einem dreistufigen Ziehprozeß mit einem handelsüblichen Pipettenziehgerät (DMZ Universalpuller, Fa. Zeitz-Instrumente, München, GER) werden Kapillaren mit einer zylindrischen Pipettenform (d.h. nicht zu einer Spitze ausgezogen) mit einem Öffnungsdurchmesser von ca. 6 µm an dem aufgeschmolzenen Ende hergestellt. Auf der anderen Seite bleibt der Ausgangsdurchmesser unverändert erhalten. Für die Reproduzierbarkeit des Transferprozesses insbesondere des Ausspülprozesses hat sich diese Pipettenform bewährt.

Wie aus Fig. 4 ersichtlich, wird die Mikrokapillare 20 in einer Spannzange 22 gehalten, die an dem Mikromanipulator 21 montiert ist, der dreidimensional im µm-Bereich positioniert werden kann und dessen Bewegung vom Bildrechner gesteuert wird. Solche Manipulatoren sind kommerziell erhältlich. Die Mikrokapillare ist über einen Schlauch 23 mit einer Spritze 24 verbunden, mit deren Hilfe der Kapillareninnendruck eingestellt wird. Der Druck wird über einen Dreiwegehahn 25 mit einem Druckmesser 26 bestimmt. Sowohl der Mikromanipulator 21 als auch die Spritze 24 werden mit Schrittmotoren, die hier nicht dargestellt sind, mit Hilfe des Bildrechners fernbedient.

Im folgenden wird der Separationsvorgang als interaktiver Prozeß beschrieben. Er kann aber auch computergesteuert vollautomatisch ablaufen.

Der gesamte Vorgang des Ansaugens und der Separation einer Bakterie (Pickvorgang) unterliegt der visuellen Kontrolle, die durch die Beobachtung mit einer 40-fachen Vergrößerung im Phasenkontrast ermöglicht wird. Von dem Mikroskop sind hier nur das Objektiv 6 und der Beleuchtungskondensor 7 dargestellt. Im Hinblick auf den erforderlichen Arbeitsabstands des Kondensors 7 von ca. 22 mm zum Objekt werden die Mikrokapillaren 20 über den Erweichungspunkt erhitzt und in der Weise umgebogen, das sie nahezu einen 90° Winkel bilden und damit platzsparend unterhalb des Kondensors positioniert werden können. Auf diese Weise kann die Beobachtung des Transferprozesses ungestört stattfinden. Für die hohe räumliche Auflösung des Transferprozesses in der Größe des Pipettendurchmessers (hier 6 µm) ist es entscheidend, daß die Pipette senkrecht auf das zu pickende Objekt trifft. Damit bildet sich kein Wasserfilm zwischen Kapillarenwand und Agarsubstrat, der umliegende Objekte beim Transferprozeß in Mitleidenschaft ziehen könnte. Zur Vorbereitung des Pickens wird über einen Unterdruck aus einem Vorratsgefäß eine Pufferlösung in die Kapillare 20 eingesogen. Hierbei reicht es aus, daß nur der verjüngte Teil der Kapillare mit der Pufferlösung gefüllt ist.

Nun wird das zu pickende Objekt bzw. die von dem Substrat 2 zu separierende Bakterie 27 durch die vom Bildrechner koordinatengesteuerte Bewegung des Mikroskopverschiebetisches 8 unterhalb der Kapillare 20 positioniert. Hierbei wird der Kapillareninnendruck auf -300 mbar gegenüber Umgebungsdruck eingestellt. Bei gleichzeitiger Mikroskopbeobachtung wird die Kapillare 20 direkt über der zu pickenden Bakterie 27 auf das Agarosesubstrat 2 aufgesetzt. Anschließend wird die Mikrokapillare 20 über die Höhenverstellung am Mikromanipulator angehoben und als Resultat bleibt im Mikroskopbild die leere Substratfläche zurück. Zum Ausspülen der Bakterie 27 wird entweder die Mikrokapillare 20 oder der Verschiebetisch 8 zu einer entsprechenden Stelle auf dem Zielsubstrat gefahren. Hierbei wird der Innendruck auf + 100 mbar erhöht. Während die Kapillare auf das Zielsubstrat (hier die Randzone 3 auf dem Substrat 2) aufgesetzt wird, findet der Ausspülprozeß statt. Nachdem die Kapillare 20 wiederum von dem Zielsubstrat 3 abgehoben wurde, wird die ausgespülte Bakterie im Phasenkontrastbild des Mikroskops erneut sichtbar.

In vier Ausführungsbeispielen wurden jeweils 10 Bakterien von einem Ausgangssubstrat auf ein Zielsubstrat, das Nährstoff enthielt, übertragen. Nach einer Anwachszeit von einigen Tagen bildeten sich in 50 bis 60 % der Fälle aus den einzelnen Bakterien Kolonien. In einem Test der Vitalitätsrate der Ausgangspopulation wurde ein Wert von 60 % bestimmt, so daß hiermit der Transferprozeß als sehr schonend angesehen werden kann. Alternativ können die Bakterien in eine Flüssigkeit z.B. PBS-Puffer ausgebracht werden. Diese Flüssigkeit kann sich in den Vertiefungen (Wells) von handelsüblichen 96- oder 384-Mikrotiterplatten befinden.

Neben dem unmittelbar nacheinander stattfindenden Pick- und Ausspülprozeß wurden auch mehrere Bakterien hintereinander gepickt und entsprechend hintereinander ausgespült. Diese Prozedur ist vergleichsweise zeitsparend, da die Wege zwischen den zu sortierenden Objekten optimiert werden können und auch die Druckeinstellung in der Kapillare jeweils nur einmal vorgenommen werden muß. Der Vorteil in der Verwendung von Bakterien besteht in der einfachen Amplifizierung durch reguläres Anwachsen. Darüber hinaus können mit Hilfe der Polymerase Chain Reaction (PCR) können aus einzeln sortierten Bakterien die Gensequenzen amplifiziert werden, die für die spezifische Eigenschaft der Bakterien verantwortlich sind.

Im folgenden wird der gesamte Selektions- und Separationsprozeß noch einmal im Überblick beschrieben. Der erste Schritt besteht in der Probenpräparation. Zu der Bakteriendispersion werden fluoreszenzmarkierte Liganden gemischt. Nach diesem Inkubationsschritt werden die Bakterien durch Abzentrifugieren mit einer Pufferlösung gewaschen. Diese Bakteriendispersion wird auf einem planaren Agarosesubstrat 2 ausgebracht. Das Substrat 2 wird auf den Verschiebetisch 8 des inversen Mikroskops 5 gebracht. Der zweite Schritt des Selektionsvorgangs besteht nun in der Durchführung der Fluoreszenzaufnahmen. Zu Beginn wird der Verschiebetisch 8 derart positioniert, daß die linke obere Ecke des abzurasternden Probenbereiches im Bildbereich des Mikroskops zu liegen kommt. Die anschließenden Tischverschiebungen folgen einem Mäander, der die gesamte Probenfläche abdeckt. Für die Fluoreszenzaufnahme wird der Laserstrahl mit einem Verschluß zur Anregung der Ligandenfluoreszenz freigegeben und in die Lichtleitfaser 10 eingekoppelt, um die Probe zu bestrahlen. Anschließend wird der Aufnahmemodus der CCD-Kamera 12 gestartet. Nach Ablauf der Belichtungszeit wird der Verschluß des Lasermoduls 9 geschlossen. Auf dem Frame Grabber Board der CCD-Kamera 12 wird das Fluoreszenzbild nach Pixeln oberhalb der gewählten Schwelle S₁ abgesucht. Falls keine Pixel gefunden werden, wird der Tisch an die nächste Position gefahren und der Bildaufnahmeprozeß wiederholt sich in der beschriebenen Weise. Werden bei der Analyse Pixel oberhalb der Schwelle detektiert, so wird eine weitere Fluoreszenzaufnahme des Bindestellenfluorophors bei der gleichen Tischposition ausgeführt. Hierzu wird der Verschluß des Lasermoduls 9 für den zweiten Laser geöffnet und die Bildaufnahme der CCD-Kamera 12 gestartet. Nach Abschluß der Bildaufnahme wird der Verschluß wieder geschlossen. Wiederum auf der Ebene des Frame Grabber Boards wird der Quotient aus dem Bild der Ligandenfluoreszenz und dem Bild der Bindestellenfluoreszenz gebildet. Aus dem Quotientenbild werden nun diejenigen Pixel detektiert, die oberhalb der Schwelle S₂ liegen. Die Pixelkoordinaten sowie die zugehörigen Quotientenwerte werden im Bildrechner bestimmt, der diese Informationen in einem Datenfile ablegt. Nach Abschluß dieser Softwareanalyse wird die nächste Tischposition angefahren und der Abrasterungsprozeß wiederholt sich zyklisch so lange, bis die gesamte Probe fluoreszenzspektroskopisch charakterisiert ist.

Ist der Abrastemngsprozeß abgeschlossen, so erfolgt nun mit der Bakterienseparation der dritte Schritt. Hierzu wird anhand der im Bildrechner gespeicherten Pickliste der Verschiebetisch sukzessive zu den Bakterien gefahren, die separiert werden sollen. Sodann wird die Mikrokapillare 20 wird auf die selektierte Bakterie 27 abgesenkt und mit Unterdruck eingesogen. Nachdem die Kapillare 20 wieder von dem Substrat 2 abgehoben worden ist, fährt der Verschiebetisch 8 zu den freigehaltenen Zielsubstratflächen 3, wo die Bakterie 27 abgesetzt wird. Dazu wird die Kapillare 20 auf das Substrat 3 abgesenkt und die Bakterie 27 wird durch den eingestellten Überdruck in der Kapillare 20 ausgespült. Dieser Prozeß wiederholt sich so lange, bis die gesamte Pickliste abgearbeitet ist. Im Bildrechner sind die Positionen der ausgebrachten Bakterien erfaßt, so daß eine spätere Zuordnung gewährleistet ist. Nach Abschluß der Bakterientransfers wird das Zielsubstrat 3 mit den separierten Bakterien vom Verschiebetisch 8 abgenommen und zur Kultivierung der Bakterien in einen Brutschrank gelegt.

In Analogie zum oben beschriebenen Ausführungsbeispiel aus dem Gebiet der molekularen Evolution kann eine Separation von Bindungsereignissen aus Bibliotheken der kombinatorischen Chemie, die sich auf Polymerbeads befinden, durchgeführt werden. Die Polymerbeads durchlaufen einen mehrstufigen chemischen Syntheseprozeß, der zu einer Molekülbibliothek führt. Diese ist dadurch gekennzeichnet, daß auf der jeweiligen Beadoberfläche eine Vielzahl gleichartiger Moleküle ausgeprägt ist, während die Molekülsorten auf unterschiedlichen Beads paarweise verschieden sind. Die Beadsuspension wird wie die Bakteriensuspension mit einem farbstoffmarkierten Liganden gemischt und anschließend gewaschen. Für die Farbstoffmarkierung wird hier vorteilhaft ein langwelliger Farbstoff wie etwa Cy5 eingesetzt, um die Hintergrundfluoresenz der Beads zu reduzieren. Anschließend werden die Beads auf eine Agarosefläche aufgebracht und fluoreszenzspektroskopisch in der oben beschriebenen Weise analysiert. Für den Separationsprozeß einzelner Beads wird als Separationsaktor ebenfalls eine Glaskapillare eingesetzt, deren Öffnungsdurchmesser nun aber den Dimensionen der Beads mit einem Durchmesser von ca. 100 µm angepaßt ist. Der Separationsprozeß wird ebenfalls durch Ansaugen einzelner Beads und anschließendem Ausspülen auf einem räumlich getrennten Substrat bewerkstelligt.

## Patentansprüche

1. Verfahren zum Screening von Molekülen aus Molekülbibliotheken bezüglich ihres individuellen Bindungsverhaltens zu mindestens einem vorgegebenen Ligand und zur Einzelselektion hochaffiner Spezies, dadurch gekennzeichnet,
a) daß die zu bindenden Liganden mit einem Fluoreszenzfarbstoff markiert werden und mit der in Form einer Suspension vorliegenden Molekülbibliothek gemischt werden
b) daß die überschüssigen, nicht an Moleküle der Molekülbibliothek gebundenen Liganden ausgewaschen und entfernt werden,
c) daß diese Mischung auf ein zweidimensionales Substrat (2) ausplattiert wird
d) daß das beschichtete Substrat (2) in ein Fluoreszenzmikroskop (5) gebracht wird und die örtlichen Fluoreszenzintensitäten auf dem Substrat (2) elektrooptisch erfasst und nach vorgegebenen Selektionskriterien in Form von Schwellwerten elektronisch diskriminiert werden, wobei die auf dem Substrat (2) befindlichen, durch eine hohe Bindungsaffinität der Liganden an Moleküle der Molekülbibliothek gekennzeichneten und damit die Selektionskriterien erfüllenden Objekte erkannt und durch Abspeicherung in einem Bildrechner lokalisiert werden
e) daß diese Objekte sequentiell durch eine vom Bildrechner koordinatengesteuerte, relative Verschiebung zwischen dem Substrat (2) und einem Separationsaktor (20,21) in den Arbeitspunkt des Separationsaktors (20,21) positioniert und durch den Separationsaktor (20,21) vom Substrat (2) entnommen und örtlich getrennt werden.

2. Verfahren nach Anspruch 1, dadurch gekennzeichnet, daß die elektrooptische Erfassung der örtlichen Fluoreszenzintensitäten auf dem Substrat (2) mit Hilfe einer CCD-Kamera (12) als Gesamtbild oder schrittweise in Form von Teilbildern erfolgt und das Gesamtbild oder die Teilbilder, gegebenenfalls nach einer Datenreduktion, dem Bildrechner zur Abspeicherung und zur bildanalytischen Weiterverarbeitung und Auswertung nach den vorgegebenen Selektionskriterien zugeleitet werden.

3. Verfahren nach Anspruch 1, dadurch gekennzeichnet, daß die elektrooptische Erfassung der örtlichen Fluoreszenzintensitäten auf dem Substrat (2) mit Hilfe eines Laser-Scanners (13,15,16) erfolgt, dessen Ausgangssignale nach den vorgegebenen Selektionskriterien elektronisch bewertet und in Verbindung mit den zugehörigen Ortskoordinaten dem Bildrechner zugeführt werden.

4. Verfahren nach Anspruch 1 bis 3, dadurch gekennzeichnet, daß zur Bereitstellung einer Molekülbibliothek als bakterielles Expressionssystem E. coli Bakterien verwendet werden.

5. Verfahren nach Anspruch 4 dadurch gekennzeichnet, daß zur Bereitstellung einer Peptid- und/oder Proteinbibliothek als spezielles bakterielles Expressionssystem E. coli Bakterien verwendet werden, die durch genetische Manipulation Variationen von LamB-Transmembranproteine exprimieren.

6. Verfahren nach Anspruch 1 bis 3, dadurch gekennzeichnet, daß zur Herstellung einer Molekülbibliothek Methoden der kombinatorischen Chemie verwendet werden und demzufolge die Molekülbibliotheken auf polymeren Beads dargestellt werden.

7. Verfahren nach Anspruch 1 bis 6, dadurch gekennzeichnet, daß verschiedene Liganden mit unterschiedlichen Fluoreszenzfarbstoffen markiert werden.

8. Verfahren nach Anspruch 7, dadurch gekennzeichnet, daß für die verschiedenen Liganden als Selektionskriterien eine Liste von Schwellwerten für die Fluoreszenzintensitäten vorgegeben wird.

9. Verfahren nach Anspruch 1 bis 6, dadurch gekennzeichnet, daß die die Molekülbibliothek tragenden Proteine auf den Bakterien oder Verbindungen aus der kombinatorischen Chemie auf Polymerbeads mit einem weiteren Fluoreszenzfarbstoff markiert werden und die Fluoreszenzintensität der Bindungsstellen als zusätzliches Selektionskriterium bei der Bildauswertung berücksichtigt wird.

10. Verfahren nach Anspruch 9, dadurch gekennzeichnet, daß das Verhältnis aus der für den Liganden charakteristischen Fluoreszenzintensität und der für die Bindungsstelle charakteristischen Fluoreszenzintensität als ein für die Bindungsaffinität maßgebliches Seiektionskriterium bei der Bildauswertung berücksichtigt wird.

11. Vorrichtung zur Durchführung des Verfahrens nach Anspruch 1 bis 8, ausgehend von einem inversen Fluoreszenzmikroskop (5) und einem Verschiebetisch (8) zur Halterung eines Trägers (2) mit der ausplattierten Objektsuspension, sowie mindestens einer Lichtquelle (9) zur Fluoreszenzanregung dadurch gekennzeichnet,
a) daß eine aus einer CCD-Kamera (12) oder aus einem Laser-Scanner (13,15,16) bestehende elektrooptische Abtasteinrichtung zur Erfassung der örtlichen Fluoreszenzintensitäten der ausplattierten Bakteriensuspension vorgesehen ist, die mit einem Bildrechner zur Weiterverarbeitung und Speicherung der Fluoreszenzbildinformation verbunden ist
b) daß oberhalb des Verschiebetisches (8) ein Separationsaktor (20,21) zum Transfer der selektierten Objekte angeordnet ist
c) und daß der Bildrechner die Positionierung des Verschiebetisches (8) relativ zum Separationsaktor (20,21) steuert.

12. Vorrichtung nach Anspruch 11, dadurch gekennzeichnet, daß der Separationsaktor (20,21) aus einer mit einem Mikromanipulator (21) verbundenen, absenkbaren Mikrokapillare (20) besteht.

13. Vorrichtung nach Anspruch 11 bis 12, dadurch gekennzeichnet, daß dem Substrat (2) das Fluoreszenzanregungslicht über mindestens einen vom Mikroskop-Strahlengang getrennten Lichtleiter (10) zugeführt wird.

14. Vorrichtung nach Anspruch 13, dadurch gekennzeichnet, daß zwei verschiedenene, in denselben Lichtleiter (10) einkoppelbare Laser mit verschiedenen Wellenlängen zur Fluoreszenzanregung vorgesehen sind.

15. Vorrichtung nach Anspruch 11 bis 14, dadurch gekennzeichnet, daß das Fluoreszenzmikroskop (5) einschließlich der Beleuchtungseinrichtung (7) und der Mikrokapillare (13) in eine Klimakammer (4) eingebaut ist, die durch einen wasserdampfgesättigten Luftstrom auf Temperaturen zwischen 1°C und 10°C gekühlt wird.
